# NEUE EUROPÄISCHE PATENTSCHRIFT

(11) **EP 2 376 413 B2**
(45) Veröffentlichungstag und Bekanntmachung des Hinweises auf die Entscheidung über den Einspruch: **31.10.2018**
(45) Hinweis auf die Patenterteilung: 20.07.2016
(21) Anmeldenummer: 09764533.7
(22) Anmeldetag: 07.12.2009
(51) Int. Cl.: C07C 29/141, C07C 31/24, C07C 31/20, C07C 31/22, C07C 29/80, C08G 18/56, C08G 63/16

(54) **VERFAHREN ZUR DESTILLATION EINES WÄSSRIGEN NEOPENTYLGLYKOLGEMISCHES**
METHOD FOR DISTILLING AN AQUEOUS MIXTURE OF NEOPENTYLGLYCOL
PROCÉDÉ DE DISTILLATION D'UN MÉLANGE AQUEUX DE NÉOPENTYLGLYCOL

(30) Priorität: 09.12.2008 EP 08171040
(43) Veröffentlichungstag der Anmeldung: 19.10.2011
(73) Patentinhaber: BASF SE, 67056 Ludwigshafen am Rhein (DE)
(72) Erfinder: SIRCH, Tilman, 67105 Schifferstadt (DE); STEINIGER, Michael, 67435 Neustadt (DE); MAAS, Steffen, 55270 Bubenheim (DE); RITTINGER, Stefan, 68199 Mannheim (DE); SCHLITTER, Stephan, Shanghai 200336 (CN)
(86) Internationale Anmeldenummer: PCT/EP2009/066522
(87) Internationale Veröffentlichungsnummer: WO 2010/066674

(56) Entgegenhaltungen:
- WO-A1-01/47847
- WO-A1-01/47848
- WO-A1-01/47849
- WO-A1-97/17313
- WO-A1-2004/013074
- WO-A1-2010/079187
- DE-A1- 10 058 303

## Beschreibung

Die Erfindung betrifft ein Verfahren zur Destillation eines wässrigen Neopentylglykolgemisches, das bei der Herstellung von Neopentylglykol aus Isobutyraldehyd und Formaldehyd erhalten wird. Weiterhin betrifft die vorliegende Erfindung auch eine Zusammensetzung enthaltend Neopentylglykol und 1 bis 10000 Gew.-ppm Neopentylglykolformiat sowie deren Verwendung.

Polymethylole, wie beispielsweise Neopentylglykol ("NPG") und Trimethyolpropan ("TMP"), werden im Kunststoffbereich zur Herstellung von Lacken, Beschichtungen, Polyurethanen und Polyestern eingesetzt.
Großtechnisch werden Polymethylole zumeist nach dem Cannizzaro-Verfahren hergestellt. Um Trimethylolpropan nach diesem Verfahren herzustellen, wird n-Butyraldehyd mit einem Überschuss Formaldehyd in Gegenwart einer anorganischen Base umgesetzt. Dabei entsteht zugleich ein Äquivalent eines anorganischen Formiats als Koppelprodukt. Die Trennung des Salzes von Trimethylolpropan ist kompliziert und erfordert zusätzlichen Aufwand. Außerdem muss das anorganische Salz - falls es nutzbringend verwertet werden soll - aufgearbeitet und gereinigt werden. Der Anfall des Koppelproduktes stellt ansonsten einen Verlust der stöchiometrisch eingesetzten Mengen an Natronlauge und Formaldehyd dar. Zusätzlich sind die Ausbeuten bei dieser anorganischen Cannizzaro-Reaktion in Bezug auf n-Butyraldehyd unbefriedigend, da im Verlauf der Reaktion hochsiedende Bestandteile gebildet werden, die nicht weiterverwertet werden können.
Ähnliche Probleme, wie für Trimethylolpropan geschildert, liegen bei der Herstellung von anderen Polymethylolen wie Trimethylolethan (aus n-Propanal und Formaldehyd) oder Trimethylolbutan (aus n-Pentanal und Formaldehyd) oder Neopentylglykol (aus Isobutyraldehyd und Formaldehyd) vor.

Zur Vermeidung dieser Nachteile wurde durch die WO 98/28253 ein mehrstufiges Verfahren zur Herstellung von Polymethylolen bekannt, bei dem Aldehyde mit 2 bis 24 C-Atomen in einer ersten Stufe (Aldolreaktion) mit Formaldehyd unter Verwendung von tertiären Aminen als Katalysator zunächst zu den entsprechenden Methylolalkanalen kondensiert und anschließend in einer weiteren Stufe (Hydrierung) zu den entsprechenden Polymethylolen hydriert werden. Dieses mehrstufige Verfahren wird üblicherweise als Hydrierverfahren bezeichnet. Dieses Verfahren ist koppelproduktarm.

Nach der ersten Stufe des Hydrierverfahrens werden nicht umgesetzte Aldehyde und ein Teil der Amin-Base im Allgemeinen von den gebildeten Methylolalkanalen destillativ abgetrennt und zurückgeführt.

Im Destillationssumpf verbleiben -- neben den gebildeten Methylolalkanalen -- Wasser, die Addukte aus Ameisensäure und den eingesetzten tertiären Aminen (Amin-Formiat) und Ameisensäure selbst.
In der Regel wird das Polymethylolalkanal nach diesen Verfahren als 20 bis 70 Gew.-%ige wässrige Lösung erhalten.

Die wässrige Polymethylolalkanal-haltige Lösung wird in einer zweiten Stufe hydriert, um die Polymethyolalkanale zu den entsprechenden Polymethylolen, wie TMP oder NPG, umzusetzen.
Bei der Hydrierung werden im Allgemeinen Nebenreaktionen beobachtet, wie eine Rückspaltungen des Methylolalkanals zu freiem Alkanal und Formaldehyd und darüber hinaus Ether-, Ester- und Acetalbildung.
Diese Nebenreaktionen führen zu einer niedrigen Hydrierselektivität und zu niedrigen Ausbeuten an Polymethylolen.
Diese Nebenprodukte können auch die die Qualität der gebildeten Polymethylole und deren Einsatz in bestimmten Anwendungen beeinträchtigen. So können sich im Endprodukt enthaltene Formiate zersetzen, wobei Ameisensäure gebildet wird. Ameisensäure kann beispielsweise die Hydrolyse von Urethan- bzw. Esterbindungen katalysieren, was zu einer beschleunigten Alterung von Lacken und Kunststoffen führt.

Im Rahmen der vorliegenden Erfindung wurde nun gefunden, dass insbesondere Mono-Ester aus Ameisensäure und der gebildeten Polymethylole (Polymethylol-Formiat) zum Formiat-Gehalt im Endprodukt beitragen.

Die Aufgabe der vorliegenden Erfindung bestand deshalb in der Bereitstellung eines Verfahrens zur Aufreinigung eines wässrigen Polymethylolgemisches, um Polymethylole mit einem geringen Gehalt an Polymethylol-Formiaten zu erhalten.

Die Aufgabe der vorliegenden Erfindung wurde gelöst durch ein Verfahren zur Destillation eines wässrigen Neopentylglykolgemisches, welches Neopentylglykol, ein tertiäres Amin, Wasser sowie das Addukt aus tertiären Amins und Ameisensäure (Amin-Formiat) enthält,
wobei man das wässrige Neopentylglykolgemisch in einer mehrstufigen Reaktion erhält, wobei man in Stufe a) Isobutyraldehyd in einer Aldolreaktion mit Formaldehyd in Gegenwart von tertiären Aminen als Katalysator zu Hydroxypivalinaldehyd kondensiert, man anschließend in Stufe b) das aus Stufe a) erhaltene Reaktionsgemisch in einen Sumpf, der überwiegend Hydroxypivalinaldehyd enthält, und einen Kopfstrom, der Leichtsieder enthält, destillativ auftrennt, und man in Stufe c) den Sumpfaustrag aus Stufe b) hydriert,
wobei das wässrige Neopentylglykolgemisch der Reaktionsaustrag aus der Hydrierung (Stufe c) ist,
das dadurch gekennzeichnet ist, dass
man das wässrige Neopentylglykolgemisch destillativ aufreinigt, in dem man Leichtsieder abtrennt,
man die Destillation in einer Destillationskolonne durchführt, die am Sumpf mit einem Verdampfer verbunden ist, wobei die Sumpftemperatur oberhalb der Verdampfungstemperatur des sich während der Destillation bildenden Mono-Esters aus Ameisensäure und Neopentylglykol (Neopentylglykolformiat) liegt,
man den Rücklauf am Kolonnenkopf so einstellt, dass man das am Kondensator anfallende Kondensat zu mehr als 30 Gew.-% in die Destillationskolonne zurückführt, und
man einen Austrag aus dem Sumpf des Verdampfers ausschleust, der überwiegend Neopentylglykol enthält.

Das in das Verfahren eingesetzte wässrige Neopentylglykolgemisch weist bevorzugt folgende Zusammensetzung auf:
20 bis 90 Gew.-% Neopentylglykol,
0 bis 5 Gew.-% Methanol,
0 bis 5 Gew.-% tertiäres Amin,
0 bis 5 Gew.% organische Nebenverbindungen,
0,01 bis 5 Gew.% des Addukts aus tertiärem Amins und Ameisensäure (Amin-Formiat), Rest Wasser.

Besonders bevorzugt hat das wässrige Neopentylglykolgemisch folgende Zusammensetzung:
50 bis 80 Gew.-% Neopentylglykol,
0,1 bis 3 Gew.-% Methanol,
0,01 bis 5 Gew.-% tertiäres Amin,
0 bis 5 Gew.% organische Nebenverbindungen,
0,01 bis 5 Gew.% des Addukts aus tertiärem Amins und Ameisensäure (Amin-Formiat), Rest Wasser.

Das wässrige Neopentylglykolgemisch erhält man in einer mehrstufigen Reaktion, wobei man in Stufe a) Isobutyraldehyd in einer Aldolreaktion mit Formaldehyd in Gegenwart von tertiären Aminen als Katalysator zu Hydroxypivalinaldehyd kondensiert, man anschließend in Stufe b) das aus Stufe a) erhaltene Reaktionsgemisch in einen Sumpf, der überwiegend Hydroxypivalinaldehyd enthält, und einen Kopfstrom, der Leichtsieder enthält, destillativ auftrennt, und man in Stufe c) den Sumpfaustrag aus Stufe b) hydriert.

Formaldehyd wird in der Regel als wässrige Formaldehydlösung in das Verfahren eingesetzt. Technisch verfügbarer Formaldehyd wird üblicherweise in wässriger Lösung in Konzentrationen von 30, 37 und 49 Gew.-% vertrieben. Es ist jedoch auch möglich Formaldehydlösungen von bis zu 60 Gew.-% in das Verfahren einzusetzen.

Technisches Formaldehyd enthält in der Regel herstellungsbedingt Ameisensäure. Die Abbauprodukte von Ameisensäure können die Standzeit des Hydrierungskatalysators in der nachfolgenden Hydrierstufe verringern, was eine Verringerung der Ausbeute an Neopentylglykol zur Folge haben kann. In einer besonderen Ausführungsform wird Formaldehyd eingesetzt, welcher einen Ameisensäuregehalt von 150 ppm oder weniger aufweist. Ein solcher Formaldehyd kann, wie in der Anmeldung PCT/EP2008/052240 (WO2008/107333) beschrieben, durch Behandlung von Formaldehyd bzw. einer wässrigen Formaldehydlösung mit basischen Ionentauschern erhalten werden. Als Anionentauscher kommen an sich bekannte, stark basische, schwach basische oder mittel basische gelförmige oder makroporöse Ionentauscher in Betracht. Dies sind zum Beispiel Anionentauscher der Struktur Polystyrolharz mit Divinylbenzol vernetzt mit tertiären Aminogruppen als funktionelle Gruppen. Auch Ionentauscher auf Basis Acrylsäure oder Methacrylsäure vernetzt mit Divinylbenzol oder Harze hergestellt durch Kondensation von Formaldehyd und Phenol kommen in Betracht. Im einzelnen kommen zum Beispiel die Handelsprodukte Ambersep® 900, Amberlyst® und Amberlite® der Firma Rohm und Haas, Philadelphia, USA sowie Lewatit® der Firma Lanxess, Leverkusen in Betracht.

Als tertiäre Amine können Amine, wie sie z. B. in DE-A 28 13 201 und DE-A 27 02 582 beschrieben sind, eingesetzt werden. Besonders bevorzugt sind Tri-n-alkylamine, insbesondere Triethylamin, Tri-n-propylamin, Tri-n-butylamin und Trimethylamin.
Ganz besonders bevorzugt sind Trimethylamin ("TMA"), Triethylamin ("TEA") und Tri-n-propylamin ("TPA"), da diese Verbindungen einen niedrigeren Siedepunkt als Neopentylglykol aufweisen und somit die destillative Entfernung aus dem Reaktionsgemisch erleichtert wird. Besonders bevorzugt wird Trimethylamin ("TMA") als tertiäres Amin in die Reaktion eingesetzt.

Die Aldolreaktion kann mit oder ohne Zusatz von organischen Lösungsmitteln oder Lösungsvermittlern durchgeführt werden. Durch den Einsatz von Lösungsmitteln, die geeignete niedrigsiedende azeotrope Gemische mit den leichtsiedenden Verbindungen bei den einzelnen Destillationen des erfindungsgemäßen Verfahrens bilden, kann gegebenenfalls der Energieaufwand bei diesen Destillationen erniedrigt und/oder die destillative Abtrennung der Leichtsieder von den hochsiedenden Verbindungen erleichtert werden.
Als Lösungsmittel sind z. B. cyclische und acyclische Ether, wie THF, Dioxan, Methyl-tert.butylether oder Alkohole, wie Methanol, Ethanol oder 2-Ethylhexanol geeignet.

Bei der Aldolreaktion beträgt das Molverhältnis von jeweils frisch zugesetztem Isobutyraldehyd zu der hinzugefügten Menge Formaldehyd zweckmäßigerweise zwischen 1:1 und 1:5, bevorzugt 1:2 bis 1:3,5.

Die Menge an bei der Aldolreaktion zugesetztem tertiärem Aminkatalysator beträgt in Bezug auf das zugesetzte Isobutyraldehyd in der Regel 0,001 bis 0,2, bevorzugt 0,01 bis 0,07 Äquivalente, d. h. das Amin wird üblicherweise in katalytischen Mengen eingesetzt.

Die Aldolreaktion wird im allgemeinen bei einer Temperatur von 5 bis 100°C, bevorzugt von 15 bis 80°C durchgeführt und die Verweilzeit wird in Abhängigkeit von der Temperatur im allgemeinen auf 0,25 bis 12 Stunden eingestellt.

Die für die Aldolreaktion beschriebenen Reaktionsführungen können bei einem Druck von im allgemeinen 1 bis 30 bar, vorzugsweise 1 bis 15 bar, besonders bevorzugt 1 bis 5 bar, zweckmäßigerweise unter dem Eigendruck des betreffenden Reaktionssystems, durchgeführt werden.

Die Aldolreaktion kann diskontinuierlich oder kontinuierlich durchgeführt werden. Vorzugsweise wird die Aldolreaktion in einem kontinuierlich betriebenen Rührkesselreaktor oder einer kontinuierlich betriebenen Rührkesselkaskade durchgeführt. Zur Einstellung der Verweilzeit kann ein Teil des Reaktionaustrags aus einem Rührkessel in den jeweiligen Rührkesselreaktor zurückgeführt werden.

Der Austrag aus der Aldolreaktion enthält üblicherweise nicht umgesetzte Ausgangsverbindungen, wie Formaldehyd, Isobutyraldehyd, sowie den eingesetzten tertiären Aminkatalysator und gegebenenfalls Wasser.

Weiterhin enthält der Austrag aus der Aldolreaktion Hydroxypivalinaldehyd, das bei der Verwendung von Isobutyraldehyd als Edukt gebildet wird.
Üblicherweise enthält der Austrag auch Verunreinigungen und Nebenprodukte aus der Aldolreaktion, wie Ameisensäure, die durch Canizzaro- oder Tischtschenko-Reaktion aus Formaldehyd entstehen können, und Formiat-Salze der eingesetzten Aminkatalysatoren, wie Trimethylammoniumformiat.

Der Austrag aus der Aldolreaktion wird anschließend üblicherweise destillativ aufgetrennt (Stufe b)).

Hierbei wird der Austrag aus der Aldolreaktion einer Destillationsvorrichtung, üblicherweise eine Kolonne, zugeführt, in der es in leichter und schwerer flüchtige Bestandteile aufgetrennt wird.
Dabei werden die Destillationsbedingungen in der Regel so gewählt, dass sich eine Fraktion aus Leichtsiedern bildet, in der als wesentliche Komponenten nicht umgesetztes Isobutyraldehyd, Formaldehyd und gegebenenfalls Wasser und Methanol enthalten sind. Diese sogenannte Leichtsiederfraktion kann in die erste Stufe des Hydrierverfahrens, der Aldolreaktion, zurückgeführt werden oder einer weiteren Aufarbeitungsstufe zugeführt werden.

Nach Abtrennung der Leichtsiederfraktion verbleibt bei der geschilderten destillativen Aufarbeitung ein schwerer flüchtiges Sumpfprodukt, das im Wesentlichen aus Hydroxypivalinaldehyd, Wasser, Ameisensäure sowie Amin-Formiat besteht.

Bei der Verwendung von TMA als tertiäres Amin, werden die Destillationsbedingungen so gewählt, dass TMA auch zum Teil in der in Leichtsiederfraktion enthalten ist und zum geringen Teil im Sumpfprodukt vorhanden ist. Bei der Verwendung von Aminen, die höher sieden als TMA, werden die Destillationsbedingungen so gewählt, dass die tertiären Amine im Sumpfprodukt angereichert werden.

Die destillative Abtrennung sollte bevorzugt bei moderatem Druck erfolgen, um nicht durch erhöhte Temperatur Hydroxypivalinaldehyd zu zersetzen. Beispielsweise kann sich Hydroxypivalinaldehyd in Hydroxypivalinsäure-Neopentylglykolester (HPN) umsetzen. Andererseits sollte der Druck nicht zu gering sein, um noch am Kopf die Leichtsieder Isobutyraldehyd, und Amin-Base, beispielsweise Trialkylamin, wie Trimethylamin, zu kondensieren.
Die Destillation sollte auch deshalb nicht bei zu niedrigem Druck stattfinden, da in der Regel unterhalb von etwa 60°C die Löslichkeit von Hydroxypivalinaldehyd (HPA) in der wässrigen Lösung schlagartig auf etwa 1 bis 3 Gew.-% abfällt, in Abhängigkeit vom Isobutyraldehyd- und Methanol-Gehalt.
Weiterhin sollte die Auftrennung des Austrags aus der Adolreaktion derart erfolgen, dass die Methanol-Menge im Leichtsiederstrom so gering wie möglich gehalten wird, damit sich die Methanolkonzentration in der Aldolreaktion nicht aufpegelt. Methanol wird in der Regel über die wässrige Formaldehyd-Lösung eingeschleppt, die je nach Herstellungsbedingungen etwa 1 bis 3 Gew.-% Methanol enthält.
Der Siedepunkt von Methanol ist niedriger als der des nicht umgesetzten Isobutyraldehyds, so dass sich Methanol am Kolonnenkopf anreichert und es somit zu einer Aufpegelung der Methanolkonzentration im Prozess kommt.
Um die Methanolkonzentration niedrig zu halten können verschiedene Maßnahmen getroffen werden.
Zum einen ist es vorteilhaft methanolarmes Formaldehyd als Edukt in die Aldolreaktion einzusetzen.

Weiterhin ist es möglich Methanol zusammen mit nicht umgesetzten Isobutyraldehyd aus dem Prozess auszuschleusen, was zu einem Verlust an Isobutyraldehyd führt.

In einer bevorzugten Ausführungsform wird die Destillation jedoch unter spezifischen Bedingungen durchgeführt, so dass Methanol ausreichend im Kolonnensumpf zurückgehalten wird. Diese bevorzugte Ausführungsform der destillativen Auftrennung des Austrags aus der Aldolreaktion ist in der Anmeldung PCT/EP2008/052240 beschrieben.

In dieser Ausführungsform wird die destillative Auftrennung in eine Leichtsiederfraktion und das Sumpfprodukt im allgemeinen bei 50 bis 200°C, vorzugsweise bei 90 bis 160°C und bei einem Druck von im allgemeinen 0,1 mbar bis 10 bar, vorzugsweise von 0,5 bis 5 bar, insbesondere bei Atmosphärendruck, in einer Destillationskolonne durchgeführt. Die Destillationskolonne wird üblicherweise bei einem Kopfdruck im Bereich von 0,5 bis 1,5 bar betrieben.

Im Kopfbereich ist bevorzugt eine zweistufige Kondensation vorgesehen, bei der die Brüden zunächst in einen bei einer Temperatur im Bereich von 50 bis 80°C betriebenen Partialkondensator geführt werden, dessen Kondensat zumindest teilweise in die Destillationskolonne zurückgeführt wird, und bei der die im Partialkondensator nicht kondensierten Brüden einem nachgeschalteten, bei einer Temperatur im Bereich von - 40 bis +30°C betriebenen Kondensator zugeführt werden, dessen Kondensat zumindest teilweise ausgeschleust wird.

Vorzugsweise wird das Kondensat des Partialkondensators zu mehr als 70 Gew.-%, besonders bevorzugt vollständig in die Destillationskolonne zurückgeführt. Dabei wird das Kondensat vorzugsweise in den Kolonnenkopf zurückgeführt. Das Kondensat des nachgeschalteten Kondensators wird vorzugsweise zu mindestens 70 Gew.-%, insbesondere vollständig ausgeschleust.

Der Partialkondensator wird bei einer Temperatur im Bereich von 50 bis 80°C, vorzugsweise 55 bis 60°C betrieben. Der nachgeschaltete Kondensator wird bei einer Temperatur im Bereich von -40 bis +30°C, vorzugsweise -10 bis +10°C betrieben. Der Kopfdruck beträgt besonders bevorzugt 1 bis 1,2 bar.

Der Sumpf der Destillationskolonne ist bevorzugt mit einem Verdampfer mit kurzer Verweilzeit verbunden, der bei einer Temperatur im Bereich von 90 bis 130°C, besonders bevorzugt von 100 bis 105°C betrieben wird. Dabei handelt es sich bei dem Verdampfer besonders bevorzugt um einen Fallfilmverdampfer, ferner können bevorzugt ein Wischfilmverdampfer oder ein Kurzwegverdampfer eingesetzt werden. Wesentlich ist dabei, dass eine kurze Verweilzeit und damit eine geringe thermische Belastung erreicht wird. Der Verdampfer kann in geeigneter Weise mit Wärme versorgt werden, beispielsweise mit 4 bar Dampf.

Bevorzugt weist die Destillationskolonne Einbauten zur Erhöhung der Trennleistung auf. Dabei wird der Reaktionsaustrag der Aldolisierung vorzugsweise in einem räumlichen Bereich zwischen 1/4 und 3/4 der theoretischen Böden der Destillationskolonne zugeführt, besonders bevorzugt in einem räumlichen Bereich zwischen 1/3 und 2/3 der theoretischen Böden der Destillationskolonne. Beispielsweise kann die Zuführung etwas oberhalb der Mitte der theoretischen Böden erfolgen (Verhältnis 3:4).
Die destillativen Einbauten können beispielsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Vorteilhaft bei der Verwendung dieser destillativen Einbauten ist der geringe Druckverlust und der geringe spezifische Flüssig-Hold-up im Vergleich zu beispielsweise Ventilböden.
Im Partialkondensator fällt als Kondensat überwiegend Wasser an, das vorzugsweise der Kolonne vollständig als Rücklauf zugeführt wird. Bei der Herstellung von Neopentylglycol (NPG) kann beispielsweise ein Gemisch als Kondensat erhalten werden, das etwa 10 Gew.-% Isobutyraldehyd, etwa 5 Gew.-% Amin-Base wie Trimethylamin, etwa 1 Gew.-% Hydroxypivalinaldehyd und etwa 5 Gew.-% Methanol neben Wasser enthält, wenn Isobutyraldehyd als Edukt verwendet wird. In diesem Falle enthalten die Restbrüden die überwiegende Menge an Isobutyraldehyd und Amin-Base wie Trimethylamin. Diese werden in dem nachgeschalteten Kondensator möglichst vollständig niedergeschlagen. Als Kühlmedium kann hierbei bevorzugt möglichst kaltes Wasser (z. B. etwa 5°C) oder eine Kältemischung (z. B. Glykol-Wasser mit beispielsweise -20°C) verwendet werden.
Vorzugsweise wird ein mit Hydroxypivalinaldehyd angereichertes Gemisch aus dem Sumpf des Verdampfers ausgeschleust. Auch ein Ausschleusen aus dem Umlauf ist möglich.

Das schwerer flüchtiges Sumpfprodukt aus der destillativen Auftrennung des Austrags aus der Aldolreaktion kann zur Verminderung der thermischen Belastung vor der weiteren Aufarbeitung in einem Kühler mit einer Kühlertemperatur im Bereich von 50 bis 80°C, besonders bevorzugt 55 bis 60°C, abgekühlt werden.

Der so erhaltene Sumpfaustrag aus Stufe b) kann nachfolgend in Stufe c) hydriert werden.

Der Sumpfaustrag aus der Stufe b) des Hydrierverfahrens enthält Hydroxypivalinaldehyd und wird in Stufe c) des Hydrierverfahrens zum Neopentylglykol hydriert ("Hydrierung").

In der Hydrierung werden vorzugsweise Katalysatoren eingesetzt, die mindestens ein Metall der Nebengruppe 8 bis 12 des Periodensystems der Elemente wie Fe, Ru, Os, Co, Rh, Ir, Ni, Pd, Pt, Cu, Ag, An, Zn, Cd, Hg, bevorzugt Fe, Co, Ni, Cu, Ru, Pd, Pt, besonders bevorzugt Cu, bevorzugt auf einem Trägermaterial enthalten.
Als Trägermaterial wird vorzugsweise ein Trägermaterial aus den Oxiden des Titans, Zirkoniums, Hafniums, Siliziums und/oder Aluminiums verwendet.

Die Herstellung der verwendbaren Katalysatoren kann nach aus dem Stand der Technik bekannten Verfahren zur Herstellung derartigen Trägerkatalysatoren erfolgen. Bevorzugt verwendet werden können auch Trägerkatalysatoren, die Kupfer auf einem Aluminiumoxid- oder Titandioxid-haltigen Trägermaterial in An- oder Abwesenheit eines oder mehrer der Elemente Magnesium, Barium, Zink oder Chrom aufweist. Derartige Katalysatoren und ihre Herstellung sind aus WO 99/44974 bekannt.
Weiterhin sind kupferhaltige Trägerkatalysatoren wie sie z.B. in WO 95/32171 beschrieben sind und die in EP-A 44 444 und DE 19 57 591 offenbarten Katalysatoren für die Hydrierung geeignet.

Die Hydrierung kann diskontinuierlich oder kontinuierlich z.B. in einem mit einer Katalysatorschüttung gefüllten Reaktorrohr durchgeführt werden, bei dem die Reaktionslösung über die Katalysatorschüttung z.B. in Riesel- oder Sumpffahrweise, wie in DE-A 19 41 633 oder DE-A 20 40 501 beschrieben, geleitet wird. Es kann vorteilhaft sein, einen Teilstrom des Reaktionsaustrages, gegebenenfalls unter Kühlung, zurückzuführen und wieder über das Katalysatorfestbett zu leiten. Ebenso kann es vorteilhaft sein, die Hydrierung in mehreren hintereinander geschalteten Reaktoren durchzuführen, beispielsweise in 2 bis 4 Reaktoren, wobei in den einzelnen Reaktoren vor dem letzten Reaktor, die Hydrierreaktion nur bis zu einem Teilumsatz von z.B. 50 bis 98 % durchgeführt wird und erst im letzten Reaktor die Hydrierung vervollständigt wird. Dabei kann es zweckmäßig sein, den Hydrieraustrag aus dem vorangehenden Reaktor vor dessen Eintritt in den nachfolgenden Reaktor zu kühlen, beispielsweise mittels Kühlvorrichtungen oder durch Eindüsen kalter Gase, wie Wasserstoff oder Stickstoff oder Einleiten eines Teilstroms an kalter Reaktionslösung.

Die Hydriertemperatur liegt im Allgemeinen zwischen 50 und 180°C, bevorzugt 90 und 140°C. Als Hydrierdruck werden im allgemeinen 10 bis 250 bar, bevorzugt 20 bis 120 bar angewandt.

Der Hydrierfeed wird in der Regel vor dem Hydrierreaktoreingang mit tertiärem Amin vermischt bis der Hydrieraustrag einen pH-Wert von 7 bis 9 aufweist. Es ist auch möglich, den Hydrierfeed und das tertiäre Amin getrennt in den Reaktor einzuspeisen und dort zu vermischen. Als tertiäre Amine können die zuvor genannten tertiären Amine, insbesondere TMA, eingesetzt werden.

Der Reaktionsaustrag aus der Hydrierung (Stufe c)) ist ein wässriges Neopentylglykolgemisch, welches Neopentylglykol, ein tertiäres Amin, Wasser sowie das Addukt aus tertiärem Amin und Ameisensäure (Amin-Formiat) enthält.

Wie obenstehend erwähnt, weist das wässrige Neopentylglykolgemisch bevorzugt folgende Zusammensetzung auf:
20 bis 90 Gew.-% Neopentylglykol,
0 bis 5 Gew.-% Methanol,
0 bis 5 Gew.-% tert. Amin,
0 bis 5 Gew.% organische Nebenverbindungen,
0,01 bis 5 Gew.% des Addukts aus tertiärem Amins und Ameisensäure (Amin-Formiat), Rest Wasser.

Besonders bevorzugt hat das wässrige Neopentylglykolgemisch folgende Zusammensetzung:
50 bis 80 Gew.-% Neopentylglykol,
0,1 bis 3 Gew.-% Methanol,
0,01 bis 5 Gew.-% tert. Amin,
0 bis 5 Gew.% organische Nebenverbindungen,
0,01 bis 5 Gew.% des Addukts aus tertiärem Amins und Ameisensäure (Amin-Formiat), Rest Wasser.

Als organische Nebenverbindung kann beispielsweise die hydrierte Form des eingesetzten Isobutyraldehyd enthalten sein, nämlich Isobutanol.

Das wässrige Neopentylglykolgemisch wird erfindungsgemäß aufgereinigt, in dem Leichtsieder von Neopentylglykol abgetrennt werden.
Die Abtrennung der Leichtsieder von dem wässrigen Neopentylglykolgemisch erfolgt erfindungsgemäß durch Destillation.

Die Destillation wird bevorzugt so durchgeführt, dass Leichtsieder, wie Wasser, Isobutanol, Methanol und tertiäres Amin im Vakuum über Kopf abgetrennt werden, insbesondere wenn das eingesetzte Amin einen niedrigeren Siedepunkt aufweist als das gebildete Neopentylglykol, wie es bei TMA, TEA und TPA der Fall ist.
Wird ein tertiäres Amin eingesetzt, welches einen höheren Siedepunkt aufweist als Neopentylglykol, so wird das tertiäre Amin zusammen mit dem Neopentylglykol am Sumpf abgetrennt und in einer nachfolgenden Destillationsstufe im Kolonnensumpf angereichert, während Neopentylglykol als Kopfprodukt abgezogen wird.

Im Rahmen der vorliegenden Erfindung wurde gefunden, dass ein Teil der Amin-Formiate während der Destillation im Kolonnensumpf oder im Abtriebsteil der Kolonne mit Neopentylglykol reagiert, unter Bildung der freien Amine und des Formiats von Neopentylglykol. Hierbei bildet sich bevorzugt der Monoester von Ameisensäure und Neopentylglykol, welcher im Rahmen dieser Offenbarung als Neopentylglykolformiat bezeichnet wird.
Die durch die Umesterungsreaktion freigesetzten Amine werden in der Regel bei der Destillation zusammen mit den anderen Leichtsiedern am Kopf der Kolonne abgetrennt.

Die Destillation sollte deshalb so geregelt werden, dass die Konzentration des gebildeten Neopentylglykolformiats im Sumpfaustrag gering gehalten wird und das Zielprodukt Neopentylglykol möglichst rein ist. Dies erfolgt erfindungsgemäß dadurch, dass man bei der Destillation eine Sumpftemperatur oberhalb der Verdampfungstemperatur des Neopentylglykolformiats wählt, so dass Neopentylglykolformiat vollständig oder weitesgehend vollständig durch Verdampfung in die Gasphase überführt werden.
Die durch die erfindungsgemäße Maßnahme bewirkte Verbesserung der Ausbeute und der Produktqualität ist wahrscheinlich darauf zurückzuführen, dass das Neopentylglykolformiat höher siedet als die anderen Leichtsieder und das Neopentylglykolformiat in der Regel deshalb im Verstärkungsteil der Kolonnen bei entsprechendem Rücklaufverhältnis niedergeschlagen werden. Das im Verstärkungsteil niedergeschlagene Neopentylglykolformiat kann mit Wasser hydrolysieren unter Rückbildung von Ameisensäure und Neopentylglykol. Die Ameisensäure wird üblicherweise am Kolonnenkopf abgetrennt, während das Neopentylglykol in der Regel aus dem Kolonnensumpf ausgetragen werden kann.

In einer bevorzugten Ausführungsform wird die Destillation folgendermaßen durchgeführt:
Der Kondensator wird in dieser bevorzugten Ausführungsform in der Regel bei einer Temperatur betrieben, in dem der überwiegende Teil der Leichtsieder bei dem entsprechenden Kopfdruck kondensiert wird.
In der Regel liegt die Betriebstemperatur des Kondensators im Bereich von 0 bis 80°C, vorzugsweise 20 bis 50°C.
Als Kühlmedium kann hierbei bevorzugt möglichst kaltes Wasser (z. B. etwa 5°C) oder eine Kältemischung (z. B. Glykol-Wasser mit beispielsweise -20°C) verwendet werden. Der Kopfdruck beträgt besonders bevorzugt 0,001 bis 0,9 bar, besonders bevorzugt 0,01 bis 0,5 bar.
Das Vakuum wird üblicherweise großtechnisch mittels eines Dampfstrahlers erzeugt. Im Kolonnensumpf wird bevorzugt eine Temperatur eingestellt, die oberhalb der Verdampfungstemperatur des Neopentylglykolformiats liegt, so dass das Neopentylglykolformiat vollständig oder weitestgehend vollständig in die Gasphase übergeht. Besonders bevorzugt wird eine Temperatur eingestellt, die 5% bis 50% über der Siedetemperatur des Neopentylglykolformiats liegt und ganz besonders bevorzugt 10% bis 20% über der Siedetemperatur des Neopentylglykolformiats liegt.
Beispielsweise kann bei der Herstellung von NPG unter Verwendung von TMA als tertiäres Amin und einen Kolonnenkopfdruck von 175 mbar, bevorzugt eine Kolonnensumpftemperatur von 150 bis 170°C, besonders bevorzugt von 160 bis 165°C eingestellt werden.
Der Rücklauf am Kolonnenkopf wird in der Regel so eingestellt, dass die überwiegende Menge des Neopentylglykolformiats in der Kolonne zurückgehalten wird. Vorzugsweise wird das am Kondensator anfallende Kondensat zu mehr als 30 Gew.-%, bevorzugt zu mehr als 60 Gew.-%, in die Destillationskolonne zurückgeführt. Dabei wird das Kondensat vorzugsweise in den Kolonnenkopf zurückgeführt.
Die für die Verdampfung erforderliche Energie wird üblicherweise durch einen Verdampfer im Kolonnensumpf eingetragen.
Dabei handelt es sich bei dem Verdampfer üblicherweise um einen Naturumlaufverdampfer oder Zwangsumlaufverdampfer. Es können aber auch Verdampfer mit kurzer Verweilzeit, Fallfilmverdampfer, Wendelrohrverdampfer, Wischfilmverdampfer oder ein Kurzwegverdampfer eingesetzt werden. Der Verdampfer kann in geeigneter Weise mit Wärme versorgt werden, beispielsweise mit 16 bar Dampf oder Wärmeträgeröl.

Bevorzugt weist die Destillationskolonne Einbauten zur Erhöhung der Trennleistung auf. Die destillativen Einbauten können beispielsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Vorteilhaft bei der Verwendung dieser destillativen Einbauten ist der geringe Druckverlust und der geringe spezifische Flüssig-Hold-up im Vergleich zu beispielsweise Ventilböden. Die Einbauten können in ein oder mehren Schüssen vorliegen.
Der Austrag aus der Hydrierung wird vorzugsweise in einem räumlichen Bereich zwischen 1/4 und 3/4 der theoretischen Böden der Destillationskolonne zugeführt, besonders bevorzugt in einem räumlichen Bereich zwischen 1/2 und 2/3 der theoretischen Böden der Destillationskolonne. Beispielsweise kann die Zuführung etwas oberhalb der Mitte der theoretischen Böden erfolgen (Verhältnis 3:4).
Die Anzahl der theoretischen Böden liegt im Allgemeinen im Bereich von 5 bis 30, vorzugsweise 10 bis 20.
Im Kondensator fällt als Kondensat ein Gemisch von Leichtsiedern an, das der Kolonne wie zuvor beschrieben, zum überwiegenden Teil als Rücklauf zugeführt wird. Beispielsweise kann das Leichtsiedergemisch Amin, Wasser sowie Isobutanol aus Isobutyraldehyd oder Methanol aus Formaldehyd enthalten.
Die nichtkondensierten Restbrüden können energetisch vorteilhaft direkt gasförmig der Verbrennung zugeführt werden, oder werden einer bei nahe Umgebungsdruck arbeitenden Destillationskolonne zugeführt. Diese nachfolgende Kolonne dient der weiteren destillativen Trennung des Kondensats.

Vorzugsweise wird ein Austrag aus dem Sumpf des Verdampfers ausgeschleust, der überwiegend Neopentylglykol enthält. Auch ein Ausschleusen aus dem Umlauf des Verdampfers ist möglich.
Der Sumpfaustrag wird im Rahmen der vorliegenden Erfindung als "Roh-Neopentylglykol" bezeichnet.

Das erfindungsgemäß erhaltene "Roh-Neopentylglykol" enthält einen im Vergleich zum Stand der Technik geringeren Anteil an Neopentylglykolformiat. Vorzugsweise beträgt der Anteil an Neopentylglykolformiat weniger als 1500 Gew.-ppm, vorzugsweise weniger als 1200 Gew.-ppm , besonders bevorzugt weniger als 800 Gew.-ppm und insbesondere bevorzugt weniger als 600 Gew.-ppm.

Das Roh-Neopentylglykol enthält weiterhin die Hydroxysäure Hydroxypivalinsäure.

Das Roh-Neopentylglykol weist bevorzugt folgende Zusammensetzung auf:
90 bis 99 Gew.-% Neopentylglykol,
0,01 bis 5 Gew.-% Hydroxypivalinsäure,
0 bis 5 Gew.% organische Nebenverbindungen.

Besonders bevorzugt hat das Roh-Polymethylol folgende Zusammensetzung:
95 bis 99 Gew.-% Neopentylglykol,
0,1 bis 2 Gew.-% Hydroxypivalinsäure,
0 bis 3 Gew.% organische Nebenverbindungen.

Um die höher siedenden, im Sumpf befindlichen, sauren Komponenten, insbesondere Hydroxypivalinsäure bei geringem Verlust an Neopentylglykol abzutrennen, wird bei der Destillation als Sumpfverdampfer in einer besonders bevorzugten Ausführungsform mindestens ein Verdampfer mit geringer Verweilzeit eingesetzt, beispielsweise ein Fallfilmverdampfer mit Rückstandsautrag, ein Dünnschichtverdampfer oder Wendelrohrverdampfer.

In einer besonderen Ausführungsform kann der Sumpf der Kolonne als eingezogener Sumpf gestaltet sein, um die Verweilzeit im Kolonnensumpf weiter zu reduzieren.

Vorzugsweise wird die Destillation des Roh-Neopentylglykol unter folgenden Bedingungen ausgeführt:
Vorteilhafterweise wird das am Kondensator anfallende Kondensat zu mehr als 30 Gew.-%, besonders bevorzugt zu mehr als 50 Gew.-% in die Destillationskolonne zurückgeführt (Rücklaufstrom). Dabei wird das Kondensat vorzugsweise in den Kolonnenkopf zurückgeführt.

Der Kondensator wird bei einer Temperatur im Bereich von 50 bis 180°C, vorzugsweise 130 bis 160°C betrieben.
Als Kühlmedium kann hierbei bevorzugt möglichst Wasser verwendet werden, das hierbei verdampft.

Der Kopfdruck beträgt besonders bevorzugt 0,001 bis 0,9 bar.
Das Vakuum wird üblicherweise großtechnisch mittels eines Dampfstrahlers erzeugt.

Die Sumpftemperatur wird in der Regel so gewählt, dass Neopentylglykol in die Gasphase überführt wird, während Hydroxypivalinsäure im Kolonnensumpf verbleibt. Bevorzugt wird eine Sumpftemperatur eingestellt, die im Bereich von 100 bis 150%, vorzugsweise 105 bis 140%, besonders bevorzugt 110 bis 130% der Siedetemperatur des Neopentylglykols liegt.
Beispielsweise kann bei der Herstellung von NPG unter Verwendung von TMA als tertiäres Amin und einen Kolonnenkopfdruck von 150 mbar, bevorzugt eine Kolonnensumpftemperatur von 150 bis 200°C, besonders bevorzugt von 160 bis 190°C eingestellt werden.

Der Sumpf der Destillationskolonne ist vorzugsweise mit mindestens einem Verdampfer mit geringer Verweilzeit verbunden.

Der Sumpf der Destillationskolonne und der Verdampfer mit geringer Verweilzeit bilden definitionsgemäß gemeinsam die Verdampfungsstufe.

Die Verweilzeit der Verdampfungsstufe wird offenbarungsgemäß berechnet, in dem das Volumen des Flüssigkeitshold-ups im heißen Teil der Kolonne (V_{Hold-up}) durch die Summe aus Rücklauf und Zulaufvolumenstrom der Kolonne dividiert wird (V_{Hold-up}/(Zulaufstrom + Rücklaufstrom)), wobei sich der Flüssigkeitshold-ups im heißen Teil der Kolonne (V_{Hold-up}) aus dem Volumen des Hold-up des Kolonnensumpfs (V_{Hold-up-Sumpf}) plus dem Volumen des Hold-up des Verdampfers (V_{Hold-up-Verdampfer}) berechnet (V_{Hold-up}=V_{Hold-up-Sumpf} + V_{Hold-up-Verdampfer}).

Die Verweilzeit in der Verdampfungsstufe beträgt vorteilhafterweise weniger als 45 Minuten, bevorzugt weniger als 30 Minuten, besonders bevorzugt weniger als 15 Minuten, insbesondere bevorzugt weniger als 10 Minuten und ganz besonders bevorzugt weniger als 5 Minuten.
Im Allgemeinen ist es bevorzugt die Verweilzeit in der Verdampfungsstufe so zu wählen, dass bei höheren Sumpftemperaturen entsprechend eine kürzere Verweilzeit eingestellt wird.

Bei einer Sumpftemperatur, die im Bereich von 130 bis 150% der Siedetemperatur des Neopentylglykols liegt, beträgt die Verweilzeit in der Verdampfungsstufe vorzugsweise 5 Minuten und weniger, besonders bevorzugt 4 Minuten und weniger und ganz besonders bevorzugt 3 Minuten und weniger

Bei einer Sumpftemperatur, die im Bereich von 120 bis 130% der Siedetemperatur des Neopentylglykols liegt, beträgt die Verweilzeit in der Verdampfungsstufe vorzugsweise 30 Minuten und weniger, besonders bevorzugt 15 Minuten und weniger und ganz besonders bevorzugt 10 Minuten und weniger und insbesondere bevorzugt 5 Minuten und weniger.

Bei einer Sumpftemperatur, die im Bereich von 100 bis 120% der Siedetemperatur des Neopentylglykols liegt, beträgt die Verweilzeit in der Verdampfungsstufe vorzugsweise 45 Minuten und weniger, besonders bevorzugt 30 Minuten und weniger und ganz besonders bevorzugt 15 Minuten und weniger und insbesondere bevorzugt 10 Minuten und weniger.

In einer weiteren besonderen Ausführungsform ist der Verdampfer mit geringer Verweilzeit mit mindestens einem weiteren Verdampfer mit geringer Verweilzeit verbunden.

Der Sumpf der Destillationskolonne und der Verdampfer mit geringer Verweilzeit bilden in dieser bevorzugten Ausführungsform definitionsgemäß gemeinsam die erste Verdampfungsstufe.
Der bzw. die weiteren Verdampfer mit geringer Verweilzeit bilden definitionsgemäß die zweite bzw. die (1+n)te (mit n≥2) Verdampfungsstufe.
Vorzugsweise ist der Verdampfer mit geringer Verweilzeit mit einem weiteren Verdampfer mit geringer Verweilzeit verbunden (zweistufige Ausführung).
In dieser Ausführungsform wird üblicherweise der überwiegende Teil der zur Verdampfung notwendigen Energie in der ersten Verdampfungsstufe eingebracht. In der zweiten Verdampferstufe kann dann die zur Verdampfung erforderliche höhere Temperatur bei einer kürzeren Verweilzeit erreicht werden, so dass die Verweilzeit in der zweiten Verdampfungsstufe geringer ist.
Die erste Stufe ist vorzugsweise als Fallfilmverdampfer oder Wendelrohrverdampfer gestaltet.
Die zweite Stufe dieser besonderen Ausführungsform ist vorzugsweise ein Fallfilmverdampfer, Wendelrohrverdampfer oder Dünnschichtverdampfer.
Die Verweilzeit in der ersten Verdampfungsstufe wird offenbarungsgemäß berechnet, in dem das Volumen des Flüssigkeitshold-ups im heißen Teil der Kolonne (V_{Hold-up}) durch die Summe aus Rücklauf und Zulaufvolumenstrom der Kolonne dividiert wird (V_{Hold-up}/(Zulaufstrom + Rücklaufstrom)), wobei sich der Flüssigkeitshold-ups im heißen Teil der Kolonne (V_{Hold-up}) aus dem Volumen des Hold-up des Kolonnensumpfs (V_{Hold-up-Sumpf}) plus dem Volumen des Hold-up des Verdampfers (V_{Hold-up-Verdampfer}) berechnet (V_{Hold-up}=V_{Hold-up-Sumpf}+ V_{Hold-up-Verdampfer}).
Die Verweilzeit der zweiten Verdampfungsstufe wird offenbarungsgemäß berechnet in dem den Flüssigkeitshold-up des zweiten Verdampfers durch den Zulaufstrom des zweiten Verdampfers dividiert wird.

Die Verweilzeit der (1+n)ten Verdampfungsstufe wird dementsprechend offenbarungsgemäß berechnet in dem den Flüssigkeitshold-up des (1+n)ten Verdampfers durch den Zulaufstrom des (1+n)ten Verdampfers dividiert wird.
In dieser bevorzugten Ausführungsform liegt die Sumpftemperatur in der ersten Verdampfungsstufe vorteilhafterweise oberhalb der Verdampfungstemperatur des Neopentylglykols.
Bevorzugt beträgt die Sumpftemperatur in der ersten Verdampfungsstufe im Bereich 100 bis 130%, besonders bevorzugt 110 bis 125% der Siedetemperatur des Neopentylglykols.
Die Temperatur im der zweiten Verdampfungsstufe wird im Allgemeinen so gewählt, dass nahezu vollständig das Neopentylglykol in die Gasphase überführt wird.
Bevorzugt beträgt die Temperatur in der zweiten Verdampfungsstufe 105 bis 150%, besonders bevorzugt 120 bis 150%, insbesondere bevorzugt 130 bis 140% der Siedetemperatur des Neopentylglykols.
Die Verweilzeit in der ersten Verdampfungsstufe beträgt vorteilhafterweise weniger als 45 Minuten, bevorzugt weniger als 30 Minuten, besonders bevorzugt weniger als 15 Minuten, insbesondere bevorzugt weniger als 10 Minuten und ganz besonders bevorzugt weniger als 5 Minuten.
Die Verweilzeit in der zweiten Verdampfungsstufe beträgt vorteilhafterweise weniger als 30 Minuten, bevorzugt weniger als 15 Minuten, besonders bevorzugt weniger als 5 Minuten, insbesondere bevorzugt weniger als 2 Minuten und ganz besonders bevorzugt weniger als 1 Minuten.
Im Allgemeinen ist es bevorzugt die Verweilzeit der Verdampfungsstufe so zu wählen, dass bei höheren Sumpftemperaturen entsprechend eine kürzere Verweilzeit eingestellt wird.
Wie voranstehend erwähnt, kann der der Verdampfer mit geringer Verweilzeit mit mehr als einem weiteren Verdampfer mit geringer Verweilzeit verbunden sein, beispielsweise mit 2 oder 3 Verdampfern, wobei der letzte der Verdampfer in der Kette die sogenannte letzte Verdampfungsstufe bildet. Die Verweilzeit und die Temperaturen in der letzten Verdampfungsstufe entsprechen den Verweilzeiten und Temperaturen der zweiten Verdampfungsstufe in der zweistufigen Ausführung.

Vorzugsweise kann bei der Herstellung von NPG unter Verwendung von TMA als tertiäres Amin in der ersten Verdampfungsstufe eine Sumpftemperatur von 135 bis 170°C, besonders bevorzugt 150 bis 160°C bei einer Verweilzeit von weniger als 45 Minuten, bevorzugt weniger als 30 Minuten eingestellt. In der zweiten Verdampfungsstufe wird vorzugsweise eine Temperatur von 160 bis 220°C, vorzugsweise 180 bis 200°C bei einer Verweilzeit von weniger als 15 Minuten, bevorzugt weniger als 10 Minuten und besonders bevorzugt weniger als 5 Minuten eingestellt.

Bevorzugt weist die Destillationskolonne Einbauten zur Erhöhung der Trennleistung auf. Die destillativen Einbauten können beispielsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Vorteilhaft bei der Verwendung dieser destillativen Einbauten ist der geringe Druckverlust und der geringe spezifische Flüssig-Hold-up im Vergleich zu beispielsweise Ventilböden. Die Einbauten können in einem oder mehreren Schüssen vorliegen.
Der Austrag aus der Hydrierung wird vorzugsweise in einem räumlichen Bereich zwischen ¼ und ¾ der theoretischen Böden der Destillationskolonne zugeführt, besonders bevorzugt in einem räumlichen Bereich zwischen 1/3 und 2/3 der theoretischen Böden der Destillationskolonne. Beispielsweise kann die Zuführung etwas oberhalb der Mitte der theoretischen Böden erfolgen (Verhältnis 3:4).
Die Anzahl der theoretischen Böden liegt im Allgemeinen im Bereich von 5 bis 30, vorzugsweise 10 bis 20.

Unter diesen Bedingungen wird in der Regel für Neopentylglykol das leichter siedende Neopentylglykol von der höher siedenden Hydroxypivalinsäure abgetrennt.

Im Kondensator fällt vorzugsweise als Kondensat aufgereinigtes Neopentylglykol an. Die Reinheit des Neopentylglykols beträgt vorzugsweise mindestens 99,0 Gew.-%, besonders bevorzugt mindestens 99,2 Gew.-%.
Die als Kondensat anfallende Zusammensetzung enthält vorzugsweise Neopentylglykol und 1 bis 10000 Gew.-ppm des Esters aus Neopentylglykol und Hydroxypivalinsäure, bevorzugt 5 bis 5000 Gew.-ppm und besonders bevorzugt 10 bis 1000 Gew.-ppm des Esters aus Neopentylglykol und Hydroxypivalinsäure.
Die als Kondensat anfallende Zusammensetzung enthält in der Regel zudem einen geringen Anteil an Neopentylglykolformiat.
Demgemäß betrifft die vorliegende Erfindung auch eine Zusammensetzung enthaltend Neopentylglykol und 1 bis 10000 Gew.-ppm Neopentylglykolformiat, vorzugsweise 5 bis 5000 Gew.-ppm und besonders bevorzugt 10 bis 1500 Gew.-ppm Neopentylglykolformiat.

Die nichtkondensierten Restbrüden enthalten in der Regel neben Leckluft und Spuren von Wasser überwiegend NPG und werden vorteilhaft direkt gasförmig in die Destillation zurückgeführt.

Vorzugsweise wird ein Austrag aus dem Sumpf des Verdampfers ausgeschleust, der überwiegend höher siedende Verbindung, wie Hydroxypivalinsäure (HPS) enthält.

Der Sumpf kann entweder thermisch in einer Verbrennung verwertet werden oder in einer nachgeschalteten Destillationskolone zugeführt werden, indem dieser in mehrere Fraktionen zerlegt wird.

Beispielsweise kann der Sumpf aus der NPG-Herstellung in eine leichtsiedende, vor allem HPS-haltige, eine mittelsiedende v.a. HPN-haltige (>97% HPN) und eine hochsiedende (v.a. Ester von HPS und HPN) Fraktion zerlegt werden.

Die Vorteile der vorliegenden Erfindung bestehen darin, dass durch das erfindungsgemäße Verfahren Neopentylglykol mit einem geringen Anteil an Neopentylglykolformiat erhalten werden. Somit eignet sich das mit dem erfindungsgemäßen Verfahren erhaltene Neopentylglykol besonders gut für den Einsatz in Polymeren oder Netzwerken, wie Lacke und Beschichtungen, in denen Bindungen durch saure Katalyse gespalten werden können, beispielsweise in Polyestern oder Polyurethanen. Das mit dem erfindungsgemäßen Verfahren erhaltene Neopentylglykol weist in diesen Anwendungen eine hohe Stabilität auf.

Ganz besonders bevorzugt kann Neopentylglykol mit einem Gehalt an Neopentylglykolformiat von weniger als 10000 Gew.-ppm, bevorzugt weniger als 5000 Gew.-ppm und besonders bevorzugt weniger als 1500 Gew.-ppm, zur Verbesserung der Hydrolysestabilität in Polymeren oder Netzwerken verwendet werden. Insbesondere können auch bevorzugt Zusammensetzung enthaltend Neopentylglykol und 1 bis 10000 Gew.-ppm Neopentylglykolformiat, vorzugsweise 5 bis 5000 Gew.-ppm und besonders bevorzugt 10 bis 1500 Gew.-ppm Neopentylglykolformiat, zur Verbesserung der Hydrolysestabilität in Polymeren oder Netzwerken verwendet werden.

Ein weiterer Vorteil des erfindungsgemäßen Verfahrens besteht darin, dass Neopentylglykol mit einer sehr hohen Ausbeute hergestellt werden kann. Dies führt insgesamt zu einer Verbesserung der Wirtschaftlichkeit des Neopentylglykol-Herstellungsprozesses.
Durch erfindungsgemäße Trennung der anfallenden Nebenprodukte und Produkte kann die Wirtschaftlichkeit des Verfahrens weiter verbessert werden, da die meisten Komponenten stofflich verwertet werden können, beispielsweise durch Rückführung in den Prozess. Der Anteil an Verbindungen, die einer Entsorgung zugeführt werden müssen, werden verringert, so dass die Entsorgungskosten bei dem erfindungsgemäßen Verfahren reduziert werden können.

Die Erfindung wird anhand der folgenden Beispiele erläutert:

### Beispiel 1:

Herstellung eines wässrigen Neopentylglykolgemisches mit dem Hydrierverfahren

### Stufe a) Aldolreaktion:

Es wurde ca. 750 g/h Isobutyraldehyd (ca. >99,5 GC-Flächen-% IBA) mit ca. 700 g/h Formaldehyd (ca. 49% Fa, 1,5% Methanol, Rest Wasser) und 80 g/h Trimethylaminlösung (50% TMA in Wasser) in einer zweistufigen Rührkesselkaskade zur Reaktion gebracht.

### Stufe b) Destillative Auftrennung des Reaktionsgemisches aus Stufe a):

Anschließend wurde die Lösung in einer Kolonne destillativ von Leichtsiedern befreit. Die Kolonne ist mit 1,5 m Gewebepackung (500m²/m³ spezifische Oberfläche) im Verstärkungsteil und 4 m Blechpackung (250 m²/m³) ausgerüstet. Der Aldolisierungsaustrag wurde oberhalb der Blechpackung zugeführt, am Kopf der Kolonne wurde ein Kondensator mit Kühlwasser (ca. 10°C) und einem nachgeschalteten Phasenscheider verwendet. Am Kopf wurde das Destillat gasförmig dem Kondensator zugeführt. Es fielen ca. 255 g/h flüssiges Kondensat an. In dem nachgeschalteten Phasenscheider wurde eine wässrige Phase von 95 g/h abgetrennt und der Kolonne vollständig zugeführt. Es wurde weiterhin vom Phasenscheider 135 g/h dem ersten Rührkessel zugeführt. Um die Regeltemperatur in der Kolonne auf 85°C halten, wurden zusätzlich der Kolonne 25 g/h organische Phase zugeführt. In der dem Kondensator nachgeschalteten Kühlfalle fielen ca. 1 g/h Flüssigkeit an (ca. 80% IBA, ca. 20% TMA), die ebenfalls rückgeführt wurden.

Die IBA-Abtrennung wurde bei einem Kopfdruck von ca. 1 bar absolut betrieben. Als Verdampfer wurde ein Fallfilmverdampfer verwendet. Es wurde eine Sumpftemperatur im Sumpf der Kolonne von 102°C eingestellt. Die Rücklaufmenge (bzw. Kühlwassermenge des Partialkondensators) zur Kolonne wurde mittels der Temperatur in der Mitte der Gewebepackung geregelt, es wurde eine Temperatur von 85°C eingestellt. Aus dem Kolonnensumpf wurde mittels einer Pumpe ca. 100 kg/h Flüssigkeit abgezogen. Diese wurde dem Fallfilmverdampfer (bestehend aus einem Ölbeheizten Edelstahlrohr, Länge, 2,5 m, Innendurchmesser ca. 21mm, Wandstärke ca. 2mm) zugeführt. Es wurde aus dem Sumpf des Fallfilmverdampfers ca. 1,5 kg/h Produkt mit einer Konzentration von ca. 0,3% Isobutyraldehyd abgezogen. Die Brüden und überschüssige Flüssigkeit wurden dem Kolonnensumpf zugeführt. Das ausgeschleuste Sumpfprodukt enthielt ca. 70 Gew.-% HPA, ca. 1,5 Gew.-% HPN, 0,3% Gew-% IBA, Rest Wasser.

### Stufe c) Hydrierung des Sumpfaustrags aus Stufe b):

Das erhaltene Sumpfprodukt wurde anschließend einer Hydrierung mittels Festbett unterzogen.

Die Aktivierung des Katalysators erfolgte folgendermaßen:
150 ml eines Cu/Al₂O₃-Katalysators wie in der EP 44444 bzw. PF 57216 beschrieben wurden in einem Rohrreaktor bei 190°C durch Überleiten eines Gemisches aus 5 Vol.-% Wasserstoff und 95 Vol.-% Stickstoff (Gesamtvolumen 50 NI/h) drucklos 24 h aktiviert.

Die Hydrierung wurde wie folgt ausgeführt:
Als Ausgangslösung diente das vorstehend als Hydrierfeed beschriebene Gemisch. Dem Gemisch wurden ca. 10 Gew.-% bezogen auf den Hydrierfeed einer 15%igen wässrigen Lösung von Trimethylamin zugesetzt. Der so erhaltene Zulauf wurde in Rieselfahrweise bei 40 bar H₂-Druck über den auf 120°C beheizten Reaktor gefahren. Die Belastung betrug 0,4 kg HPA/ (I_{Kat.}*h). Ein Teil des Hydrieraustrags wurde dem Zulauf wieder zugemischt (Kreislauffahrweise). Das Verhältnis von Kreislauf zu Zulauf betrug 10:1. Der pH-Wert wurde von Proben des Reaktoraustrags bei Raumtemperatur zu 8,9 gemessen.

Die Zusammensetzung des wässrigen Neopentylglykolgemisches aus Stufe c) beträgt:
NPG: 69 Gew.-%
Methanol: 3,5 Gew.-%
TMA: 2 Gew.-%
organische Nebenverbindungen (HPS, Isobutanol): <2 Gew.-%
TMA-Formiat: 1 Gew.-%
Wasser: 23 Gew.-%

### Beispiel 2: Destillation eines wässrigen Neopentylglykolgemisches aus Stufe c)

Der aus der Hydroxypivalinaldehyd-Hydrierung erhaltene Austrag wurde einer destillativen Trennung zugeführt. Es wurde eine Packungskolonne, (DN 50 mm) mit drei Sequenzen von strukturierter Packung mit je 1 m Länge und 500 m²/m³ spezifischer Oberfläche verwendet. Der Zulauf erfolgt oberhalb der untersten Sequenz. Es wurde ein Kopfdruck von. ca. 175 mbar absolut eingestellt. Im Sumpf wurde eine Temperatur von 164°C eingestellt. Der Wert von 164°C entspricht 110% des Siedepunktes von NPG-Formiat bei 175 mbar.

Die Energie wurde der Kolonne mittels eines Naturumlaufverdampfers zugeführt, es kann aber auch ein anderer Verdampfer, z.B. Fallfilmverdampfer eingesetzt werden. Die am Kopf erhaltenen Brüden wurden einem Kondensator zugeführt, dieser schlägt bei 30°C die erhaltenen Brüden nahezu vollständig nieder. Das Vakuum wurde über einer einfachen handelsüblichen Wasserstrahlvakuumpumpe erzeugt. Vom erhaltenen Destillat wurden ca. 350 g/h ausgeschleust, ca. 250 g/h werden der Kolonne auf dem obersten Packungsabschnitt als Rücklauf zudosiert. Das zur Vakuumerzeugung verwendete Wasser wurde einer biologischen Abwasserbehandlung zugeführt.

Im Sumpf wurde ein Roh-NPG mit folgender Zusammensetzung erhalten:
97 Gew.-% NPG mit ca. 400 Gew.-ppm NPG-Formiat.

### Vergleichsbeispiel:

Bei sonst gleichen Bedingungen wie in Beispiel 2 wurde die Aufkochrate im Sumpf durch Reduktion des Rücklaufs auf 75 g/h reduziert. Im Sumpf wurde eine Temperatur von 145°C eingestellt. Im Sumpf wurde ein Roh-NPG mit folgender Zusammensetzung erhalten:
97 Gew.-% NPG mit ca. 5700 Gew.-ppm NPG-Formiat.

### Beispiel 3: Destillation eines wässrigen Neopentylglykolgemisches aus Stufe c)

Der aus der Hydroxypivalinaldehyd-Hydrierung erhaltene Austrag wurde einer destillativen Trennung zugeführt. Es wurde eine Packungskolonne, (DN 50 mm) mit drei Sequenzen von strukturierter Packung mit je 1 m Länge und 500 m²/m³ spezifischer Oberfläche verwendet. Der Zulauf erfolgt oberhalb der untersten Sequenz. Es wurde ein Kopfdruck von. ca. 70 mbar absolut eingestellt. Im Sumpf wurde eine Temperatur von 148°C eingestellt. Der Wert von 148°C entspricht 120% des Siedepunktes von NPG-Formiat bei 70 mbar.

Die Energie wurde der Kolonne mittels eines Naturumlaufverdampfers zugeführt, es kann aber auch ein anderer Verdampfer, z.B. Fallfilmverdampfer eingesetzt werden. Die am Kopf erhaltenen Brüden wurden einem Kondensator zugeführt, dieser schlägt bei 10°C die erhaltenen Brüden nahezu vollständig nieder. Das Vakuum wurde über einer einfachen handelsüblichen Wasserstrahlvakuumpumpe erzeugt. Vom erhaltenen Destillat wurden ca. 350 g/h ausgeschleust, ca. 250 g/h werden der Kolonne auf dem obersten Packungsabschnitt als Rücklauf zudosiert. Das zur Vakuumerzeugung verwendete Wasser wurde einer biologischen Abwasserbehandlung zugeführt.

Im Sumpf wurde ein Roh-NPG mit folgender Zusammensetzung erhalten: 97 Gew.-% NPG mit ca. 400 Gew.-ppm NPG-Formiat.

### Beispiel 4: Destillation eines wässrigen Neopentylglykolgemisches aus Stufe c)

Der aus der Hydroxypivalinaldehyd-Hydrierung erhaltene Austrag wurde einer destillativen Trennung zugeführt. Es wurde eine Packungskolonne, (DN 50 mm) mit drei Sequenzen von strukturierter Packung mit je 1 m Länge und 500 m²/m³ spezifischer Oberfläche verwendet. Der Zulauf erfolgt oberhalb der untersten Sequenz. Es wurde ein Kopfdruck von. ca. 500 mbar absolut eingestellt. Im Sumpf wurde eine Temperatur von 189°C eingestellt. Der Wert von 189°C entspricht 106% des Siedepunktes von NPG-Formiat bei 500 mbar.

Die Energie wurde der Kolonne mittels eines Naturumlaufverdampfers zugeführt, es kann aber auch ein anderer Verdampfer, z.B. Fallfilmverdampfer eingesetzt werden. Die am Kopf erhaltenen Brüden wurden einem Kondensator zugeführt, dieser schlägt bei 50°C die erhaltenen Brüden nahezu vollständig nieder. Das Vakuum wurde über einer einfachen handelsüblichen Wasserstrahlvakuumpumpe erzeugt. Vom erhaltenen Destillat wurden ca. 350 g/h ausgeschleust, ca. 250 g/h werden der Kolonne auf dem obersten Packungsabschnitt als Rücklauf zudosiert. Das zur Vakuumerzeugung verwendete Wasser wurde einer biologischen Abwasserbehandlung zugeführt.

Im Sumpf wurde ein Roh-NPG mit folgender Zusammensetzung erhalten:
97 Gew.-% NPG mit ca. 400 Gew.-ppm NPG-Formiat.

## Patentansprüche

1. Verfahren zur Destillation eines wässrigen Neopentylglykolgemisches, welches Neopentylglykol, ein tertiäres Amin, Wasser sowie das Addukt aus tertiärem Amin und Ameisensäure (Amin-Formiat) enthält,
wobei man das wässrige Neopentylglykolgemisch in einer mehrstufigen Reaktion erhält, wobei man in Stufe a) Isobutyraldehyd in einer Aldolreaktion mit Formaldehyd in Gegenwart von tertiären Aminen als Katalysator zu Hydroxypivalinaldehyd kondensiert, man anschließend in Stufe b) das aus Stufe a) erhaltene Reaktionsgemisch in einen Sumpf, der überwiegend Hydroxypivalinaldehyd enthält, und einen Kopfstrom, der Leichtsieder enthält, destillativ auftrennt, und man in Stufe c) den Sumpfaustrag aus Stufe b) hydriert,
wobei das wässrige Neopentylglykolgemisch der Reaktionsaustrag aus der Hydrierung (Stufe c) ist,
**dadurch gekennzeichnet, dass**
man das wässrige Neopentylglykolgemisch destillativ aufreinigt, in dem man Leichtsieder abtrennt,
man die Destillation in einer Destillationskolonne durchführt, die am Sumpf mit einem Verdampfer verbunden ist, wobei die Sumpftemperatur oberhalb der Verdampfungstemperatur des sich während der Destillation bildenden Mono-Esters aus Ameisensäure und Neopentylglykol (Neopentylglykolformiat) liegt,
man den Rücklauf am Kolonnenkopf so einstellt, dass man das am Kondensator anfallende Kondensat zu mehr als 30 Gew.-% in die Destillationskolonne zurückführt, und
man einen Austrag aus dem Sumpf des Verdampfers ausschleust, der überwiegend Neopentylglykol enthält.

2. Verfahren nach Anspruch 1, **dadurch gekennzeichnet, dass** das die Zusammensetzung des wässrigen Neopentylglykolgemisches
20 bis 90 Gew.-% Neopentylglykol,
0 bis 5 Gew.-% Methanol,
0 bis 5 Gew.-% tertiäres Amin,
0 bis 5 Gew.% organische Nebenverbindungen,
0,01 bis 5 Gew.% des Addukts aus tertiärem Amins und Ameisensäure (Amin-Formiat), Rest Wasser,
enthält.

3. Verfahren nach mindestens einem der Ansprüche 1 bis 2, **dadurch gekennzeichnet, dass** das tertiäre Amin Triethylamin, Tri-n-propylamin, Tri-n-butylamin oder Trimethylamin ist.

4. Verfahren nach Anspruch 3, **dadurch gekennzeichnet, dass** das tertiäre Amin Trimethylamin ist.

5. Verfahren nach mindestens einem der Ansprüche 1 bis 4, **dadurch gekennzeichnet, dass** der Druck am Kolonnenkopf 0,001 bis 0,9 bar beträgt und die Betriebstemperatur des Kondensators im Bereich von 0 bis 80°C liegt.

6. Verfahren nach mindestens einem der Ansprüche 1 bis 5, **dadurch gekennzeichnet, dass** im Kolonnensumpf eine Temperatur eingestellt wird, die 10% bis 50% über der Siedetemperatur des Neopentylglykolformiats liegt.

7. Verfahren nach mindestens einem der Ansprüche 1 bis 6, **dadurch gekennzeichnet, dass** das am Kondensator anfallende Kondensat zu mehr als 60 Gew.-% in die Destillationskolonne zurückgeführt wird.

8. Verfahren nach mindestens einem der Ansprüche 1 bis 7, **dadurch gekennzeichnet, dass** die Destillationskolonne 5 bis 30 theoretische Böden aufweist.

9. Verfahren nach mindestens einem der Ansprüche 1 bis 8, **dadurch gekennzeichnet, dass** die Destillationskolonne Einbauten zur Erhöhung der Trennleistung aufweist und das wässrige Neopentylglykolgemisch in einem räumlichen Bereich zwischen ¼ und ¾ der theoretischen Böden der Destillationskolonne zugeführt wird.

10. Verfahren nach mindestens einem der Ansprüche 1 bis 9, **dadurch gekennzeichnet, dass** man dem Sumpfaustrag aus Stufe b) tertiäres Amin zugibt.

11. Verfahren nach mindestens einem der Ansprüche 1 bis 10, **dadurch gekennzeichnet, dass** der Sumpfaustrag aus der Destillation der wässrigem Neopentylglykolgemisches in einen Kopfstrom, enthaltend Neopentylglykol, und einen Sumpfstrom, enthaltend höher siedende organische Komponenten, aufgetrennt wird.

## Claims

1. A process for distilling an aqueous neopentyl glycol mixture which comprises neopentyl glycol, a tertiary amine, water and the adduct of tertiary amine and formic acid (amine formate),
where the aqueous neopentyl glycol mixture is obtained in a multistage reaction involving, in stage a) condensing isobutyraldehyde in an aldol reaction with formaldehyde in the presence of tertiary amines as a catalyst to give hydroxypivalaldehyde, then, in stage b), distillatively separating the reaction mixture obtained from stage a) into bottoms comprising predominantly hydroxypivalaldehyde and a top stream which comprises low boilers, and, in stage c), hydrogenating the bottoms discharge from stage b), where the aqueous neopentyl glycol mixture is the reaction discharge from the hydrogenation (stage c), which comprises
the aqueous neopentyl glycol mixture is subjected to distillative purification by removing low boilers performing the distillation in a distillation column which is connected at the bottom to an evaporator, the bottom temperature being above the evaporation temperature of the monester of formic acid and neopentyl glycol (neopentyl glycol formate) which forms during the distillation,
the reflux at the column top is adjusted such that the more than 30% by weight of the condensate accumulating at the condenser is recycled into the distillation column
and an output from the bottoms of the evaporator containing predominantly neopentyl glycol is discharged.

2. The process according to claim 1, wherein the composition of the aqueous neopentyl glycol mixture comprises
20 to 90% by weight of neopentyl glycol,
0 to 5% by weight of methanol,
0 to 5% by weight of tertiary amine,
0 to 5% by weight of organic secondary compounds,
0.01 to 5% by weight of the adduct of tertiary amine and formic acid (amine formate), remainder water.

3. The process according to at least one of claims 1 and 2, wherein the tertiary amine is triethylamine, tri-n-propylamine, tri-n-butylamine or trimethylamine.

4. The process according to claim 3, wherein the tertiary amine is trimethylamine.

5. The process according to at least one of claims 1 to 4, wherein the pressure at the top of the column is 0.001 to 0.9 bar and the operating temperature of the condenser is in the range from 0 to 80°C.

6. The process according to at least one of claims 1 to 5, wherein a temperature which is 10% to 50% above the boiling temperature of the neopentyl glycol formate is established in the column bottom.

7. The process according to at least one of claims 1 to 6, wherein condensate obtained in the condenser is recycled into the distillation column to an extent of more than 60% by weight.

8. The process according to at least one of claims 1 to 7, wherein the distillation column has 5 to 30 theoretical plates.

9. The process according to at least one of claims 1 to 8, wherein the distillation column has internals for increasing the separating performance and the aqueous neopentyl glycol mixture is fed in within a spatial region between ¼ and ¾ of the theoretical plates of the distillation column.

10. The process according to at least one of claims 1 to 9, wherein tertiary amine is added to the bottoms discharge from stage b).

11. The process according to at least one of claims 1 to 10, wherein the bottoms discharge from the distillation of the aqueous neopentyl glycol mixture is separated into a top stream comprising neopentyl glycol, and a bottom stream comprising higher-boiling organic components.

## Revendications

1. Procédé de distillation d'un mélange aqueux du néopentylglycol, qui contient de néopentylglycol, une amine tertiaire, de l'eau et l'adduit d'une amine tertiaire et d'acide formique (formiate d'amine),
le mélange aqueux de néopentylglycol étant obtenu par une réaction à plusieurs étapes, selon laquelle à l'étape a), de l'isobutyraldéhyde est condensé par une réaction d'aldolisation avec du formaldéhyde en présence d'amines tertiaires en tant que catalyseur en hydroxypiralinaldéhyde puis à l'étape b), le mélange réactionnel obtenu à l'étape a) est séparé par distillation en un fond, qui contient principalement hydroxypiralinaldéhyde, et un courant de tête, qui contient les composants de point d'ébullition faible, et la sortie de fond de l'étape b) est hydrogénée à l'étape c), le mélange aqueux de néopentylglycol étant la sortie de réaction de l'hydrogénation (étape c), **caractérisé en ce que**
le mélange aqueux de néopentylglycol est purifié par distillation, en séparant les composants de point d'ébullition faible,
la distillation est réalisée dans une colonne de distillation, qui est reliée au fond avec un évaporateur, la température de fond étant supérieure à la température d'évaporation du monoester qui se forme pendant la distillation à partir d'acide formique et de néopentylglycol (formiate de néopentylglycol),
le reflux à la tête de la colonne est ajusté de telle sorte que le condensat formé dans le condensateur soit recyclé à hauteur de plus de 30 % en poids dans la colonne de distillation, et une sortie du fond de l'évaporateur est évacuée, qui contient principalement du néopentylglycol.

2. Procédé selon la revendication 1, **caractérisé en ce que** la composition du mélange aqueux de néopentylglycol contient
20 à 90 % en poids de néopentylglycol,
0 à 5 % en poids de méthanol,
0 à 5 % en poids d'une amine tertiaire,
0 à 5 % en poids de composés secondaires organiques,
0,01 à 5 % en poids de l'adduit d'une amine tertiaire et d'acide formique (formiate d'amine),
le reste étant de l'eau.

3. Procédé selon au moins l'une quelconque des revendications 1 et 2, **caractérisé en ce que** l'amine tertiaire est la triéthylamine, la tri-n-propylamine, la tri-n-butylamine ou la triméthylamine.

4. Procédé selon la revendication 3, **caractérisé en ce que** l'amine tertiaire est la triméthylamine.

5. Procédé selon au moins l'une quelconque des revendications 1 à 4, **caractérisé en ce que** la pression à la tête de la colonne est de 0,001 à 0,9 bar et la température d'exploitation du condensateur se situe dans la plage allant de 0 à 80 °C.

6. Procédé selon au moins l'une quelconque des revendications 1 à 5, **caractérisé en ce qu'**une température qui se situe 10 % à 50 % au-dessus de la température d'ébullition du formiate de néopentylglycol est ajustée dans le fond de la colonne.

7. Procédé selon au moins l'une quelconque des revendications 1 à 6, **caractérisé en ce que** le condensé formé dans le condensateur est recyclé à plus de 60 % dans la colonne de distillation.

8. Procédé selon au moins l'une quelconque des revendications 1 à 7, **caractérisé en ce que** la colonne de distillation comprend 5 à 30 plateaux théoriques.

9. Procédé selon au moins l'une quelconque des revendications 1 à 8, **caractérisé en ce que** la colonne de distillation comprend des composants internes pour augmenter le pouvoir de séparation, et le mélange aqueux de néopentylglycol est introduit dans une zone de l'espace comprise entre ¼ et ¾ des plateaux théoriques de la colonne de distillation.

10. Procédé selon au moins l'une quelconque des revendications 1 à 9, **caractérisé en ce qu'**une amine tertiaire est ajoutée à la sortie de fond de l'étape b) .

11. Procédé selon au moins l'une quelconque des revendications 1 à 10, **caractérisé en ce que** la sortie de fond de la distillation du mélange aqueux de néopentylglycol est séparée en un courant de tête, contenant le néopentylglycol, et un courant de fond, contenant les composants organiques de point d'ébullition plus élevé.
